# EUROPEAN PATENT SPECIFICATION

(11) **EP 3 169 795 B1**
(45) Date of publication and mention of the grant of the patent: **20.11.2019**
(21) Application number: 15822198.6
(22) Date of filing: 17.07.2015
(51) Int. Cl.: C12Q 1/37, G01N 21/78, G01N 33/52, G01N 33/58

(54) **PREPARATION OF STABILIZER-FREE GOLD SOLS AND THEIR CONFIGURATION INTO A GELATIN-COATED PROTEASE-INDICATING HOMOGENEOUS (ONE STEP) ASSAY**
HERSTELLUNG VON STABILISATORFREIEN GOLDSOLEN UND DEREN KONFIGURATION IN EINEN GELATINEBESCHICHTETEN HOMOGENEN (EINSTUFIGEN) TEST ZUR ANZEIGE VON PROTEASE
PRÉPARATION DE SOLS D'OR EXEMPTS DE STABILISATEUR ET LEUR CONFIGURATION EN UN DOSAGE HOMOGÈNE (EN UNE ÉTAPE) INDICATEUR DE PROTÉASE REVÊTU DE GÉLATINE

(30) Priority: 17.07.2014 US 201461999088 P; 06.03.2015 US 201562177037 P; 13.04.2015 US 201562178475 P
(43) Date of publication of application: 24.05.2017
(73) Proprietor: Sano LLC, Wellesley, MA 02481 (US)
(72) Inventor: HAYRE, Paul N., Wellesley, MA 02481 (US); KERSCHENSTEINER, Daniel, A., Kernersville, NC 27284 (US)
(74) Representative: Wallinger, Michael
(86) International application number: PCT/US2015/040859
(87) International publication number: WO 2016/011323

(56) References cited:
- WO-A1-2005/085854
- WO-A2-2009/082445
- US-A- 5 310 647
- US-A1- 2003 119 073
- US-A1- 2014 004 546
- CRISTIAN GUARISE ET AL: "Gold nanoparticles-based protease assay", PROCEEDINGS NATIONAL ACADEMY OF SCIENCES PNAS, NATIONAL ACADEMY OF SCIENCES, US, vol. 103, no. 11, 14 March 2006 (2006-03-14), pages 3978-3982, XP002651288, ISSN: 0027-8424, DOI: 10.1073/PNAS.0509372103 [retrieved on 2006-03-01]
- ANDREESCU, D ET AL.: 'Stabilizer-Free Nanosized Gold Sols' J. COLLOID INTERFACE SCI. vol. 298, no. 2, 2006, pages 742 - 751, XP024909561

## Description

### TECHNICAL FIELD

The present invention is in the technical field of enzyme detection. More particularly, the present invention is in the technical field of diagnostics. More particularly, the present invention is in the technical field of detecting the presence of proteases by means of a one-step procedure.

The present invention is in the technical field of gold sols. More particularly, the present invention is in the technical field of the preparation of stabilizer-free (uncapped) gold sols at room temperature. More particularly, the present invention is in the technical field of gelatin protection of stabilizer-free gold sol. More particularly, the present invention is in the technical field of proteolysis by hydrolytic enzymes, where proteolysis of the gelatin protective coating results in a destabilization of gold sol. More particularly, the present invention is in the technical field of gold sol flocculation with a color change as a result of proteolysis by the proteases indicating their presence.

### BACKGROUND ART

### Gold Dispersions

Due to its presence in a readily accessible pure form in nature, esthetically pleasing properties, and exceptional resistance to tarnishing and corrosion, gold has captured the attention of both primitive and modern societies. While the bulk metal itself can be used successfully in various applications, dispersions of fine gold particles are, by far, more important for both scientific and practical applications. The interest in such dispersions can be traced back to ancient times when their preparation and use for decorative and therapeutic purposes was quite common.

Faraday's gold sols, produced in the mid-nineteenth century, resulted from the first documented scientific investigation of dispersions of fine particles of this metal. Since then, the preoccupation with these systems has progressively escalated, fueled primarily by their fascinating properties (especially optical) and their potential for new applications in catalysis, nonlinear optics, electronics, pigments, biology, sensors, biosensors, dentistry, heat reflecting coatings, and other domains of high technology and medicine.

Since Faraday's pioneering work, many different routes to produce colloidal gold have been reported, including the condensation of metal vapors in solvents, in polymers or other matrices, as well as the thermal decomposition of precursor metallic compounds suspended in either liquids or gas streams (aerosol thermolysis). The vast majority of the processes, however, involve the reduction of gold compounds in solutions. The electrons needed in the latter process can be either generated 'in situ,' as for example in cases where HAuCl₄ solutions are subjected to y rays, pulse radiolysis, ultraviolet, or visible radiation, or can be supplied by various reducing chemical species present in solution. Since the redox potential of gold is high, the list of compounds capable of serving as reducing agents has in a striking similarity with the difference in the behavior between saturated and unsaturated alcohols.

Despite their desirable reducing properties, these compounds have been used in the case of gold mostly for analytical determinations, metal recovery, electroless plating, and only a few attempts were made to produce stable dispersions of gold particles. In the vast majority of the precipitation processes, especially in concentrated solutions and non-aqueous media, the addition of protective species is necessary in order to obtain stable gold colloids. Being readily available, natural polymers (gelatin, dextrin) and polyelectrolytes (gums) were historically the first and probably the most frequently used additives for this purpose. Large molecules of water-soluble synthetic polyelectrolytes (polycarbonates, polysulfonates, and polyphosphates) and polymers (polyvinyl alcohol, polyvinylpyrrolidone, polyacrylates, and polyacrylamides) can also be equally effective in stabilizing aqueous metal dispersions and can be often tailored to specific experimental conditions.

While the use of such additives greatly improves the ability to generate stable dispersions of gold particles, in many cases the presence of these compounds on the surface of the metal affects the physical and chemical properties of the solid surface in undesirable ways. For this reason, there is a significant interest in the preparation of electrostatically stabilized gold colloids in which the surface of the metal is amenable to various subsequent physical and chemical processes.

Probably the most well-known and thoroughly investigated example of such a system is the colloidal gold dispersion obtained by the reduction of tetrachloroauric acid with sodium citrate. The method pioneered by J. Turkevich et al. in 1951 and refined by G. Frens in the 1970s, is the simplest one available. In general, it is used to produce modestly monodisperse spherical gold nanoparticles suspended in water of around 10-20 nm in diameter. Larger particles can be produced, but this comes at the cost of monodispersity and shape. It involves the reaction of small amounts of hot chloroauric acid with small amounts of sodium citrate solution. The colloidal gold will form because the citrate ions act as both a reducing agent and a capping agent. To produce larger particles, less sodium citrate should be added (possibly down to 0.05%, after which there simply would not be enough to reduce all the gold). The reduction in the amount of sodium citrate will reduce the amount of the citrate ions available for stabilizing the particles, and this will cause the small particles to aggregate into bigger ones (until the total surface area of all particles becomes small enough to be covered by the existing citrate ions).

In this case, the excess citrate ions adsorbed on the surface of the metallic particles provide a high enough surface charge to prevent the aggregation during the precipitation process. However, because of the fact that citric acid is a rather mild reducing agent, the reduction reaction must be conducted at elevated temperature (70-80°C) and may take up to an hour.

Alternatively, a colloidal gold dispersion may be made by reducing tetrachloroauric acid with iso-ascorbate at room temperature (approximately 20°C), instead of with sodium citrate at higher temperatures. Andreescu and coworkers (Journal of Colloid and Interface Science 298 (2006) 742-751) describe the following overall reduction reaction:

2HAuCl₄ + 3C₆H₈O₆ → 2Au⁰ + 3C₆H₆O₆ + 8HCl.

### Detection of Proteases

A necessary part of wound healing is the proteolysis of extracellular matrix (ECM) and its sloughing off. This involves the response of many proteases including the gelatinases. A major component of ECM and of the dermis is collagen. Gelatinases are enzymes that have specific high affinity for and catalyze the hydrolysis of gelatin, a denatured or melted form of collagen. As such, gelatinases are important in many disease processes and detecting and measuring them can be a guide to discover diseases. Diseases as diverse as the following involve gelatinases: metastases (cancer) can be noninvasively detected by identifying selected gelatinases (matrix metalloproteases MMP-2 and MMP-9) in the urine; a condition of the eye known as ocular rosacea, which is when excessive amounts of MMPs are found in the eye which if untreated may lead to blindness; periodontal disease in which excessive amounts of gelatinases and other molecules destroy the periodontal ligament which holds the tooth intact; non-healing wounds are acute wounds which have excessive gelatinase and become chronic; excessive gelatinase can be found in the synovial fluid of animals suffering from osteoarthritis and can lead to lameness.

It is important to measure and detect these enzymes as rapidly as possible and at the point-of-care so therapy can begin as quickly as possible. Some tests to detect protease have used gelatin zymography, immunochromatography, and enzyme substrate analogues to detect these enzymes.

Analyzing the presence of proteases in a biological sample traditionally has involved studies using several different techniques that use antibodies to detect and measure levels of proteases, e.g., enzyme-linked immunosorbent assays (ELISAs) or lateral flow tests (LF). Other assays that measure the enzymatic activity of proteases employ specific chromogenic substrate analogues. Gelatin zymography is a technique that does not require antibodies; however it requires specialized equipment and is laborious. All of the aforementioned techniques are labor-intensive and time-consuming, requiring specialized equipment and skill.

For comparison purposes, the following table lists some of the typical assays available that are capable of measuring proteases in wounds in the laboratory. They include traditional blue azo-dyed insoluble collagen (Azocoll, Sigma); a microtiter plate ELISA test using a MMP-9 monoclonal antibody (Invitrogen); a synthetic fluorescent substrate mimicking the target region of MMPs (Sigma); and analytical gelatin zymography:

| **Azocoll** | | **MMP-9 ELISA** | **Mca--NH2*** | **Gelatin Zymography** |
|---|---|---|---|---|
| **Sensitivity** | µg | 10 pg | 10 pg | 10 pg |
| **Specificity** | Gelatinases (MMP-2,-9) | Gelatinase MMP-9 | All MMPs | Gelatinase lysing |
| **Cost** | $ | $$ ($750/96 tests) | $$ | $$$ |
| **Time of Analysis** | 6h, 37°C | 4h, 37°C | 2h, 37°C | 3-5 Days, cold room |

| | | | | |
|---|---|---|---|---|
| ***Mca- Lys-Pro-Len-Gly-Leu-Dpa-Ala-Arg-NH2** | | | | |

Wound ChekTM (Systagenix) is an immunochromatogenic test for inflammatory proteases in wounds. At a cost of $30/test, it takes 20 minutes in a series of timed procedures at room temperature. It is not available in the US. There is no public data available on its sensitivity and specificity. Since proteases self-digest, immunochromatographic tests cannot discriminate between active and inactive proteases.

### SUMMARY OF THE INVENTION

The present invention solves the above-mentioned problems by using stabilizer-free and room temperature-prepared nanoparticles of gold sol adsorbed with gelatin and combined with CaCl₂ to prepare a colorimetric homogeneous (one step requiring no separation) protease indicator test. A wide variety of proteases react with the gelatin coating, inducing a destabilization of the gold sol, then the Ca ions produce an aggregation of the gold sol which results in a color change to grey or black, which is visible to the eye. This allows for an assay to detect protease activity.

### BRIEF DESCRIPTION OF THE DRAWINGS

Figure 1 is an image showing a color change resulting from exposure of gelatin-coated nanoparticles to protease hydrolysis.
Figures 2A and 2B are graphs showing turbidity versus time (Fig. 2A) and the initial rate of turbidity versus trypsin concentration (Fig. 2B) in an exemplary embodiment of exposing gelatin-coated gold nanoparticles to trypsin.
Figure 3 is an image showing color changes of assay samples having different concentrations of trypsin.
Figure 4 is a graph of turbidity versus trypsin concentration for the assay samples of Fig. 3.
Figure 5 is an image showing color changes of assay samples having different concentrations of trypsin, in 0.5 ml sample volumes.
Figure 6 is an image showing color changes of assay samples having different concentrations of trypsin, in 0.25 ml sample volumes.
Figure 7 is a graph of detection limit versus assay sample volume, combining data from Figs. 3-6.
Figure 8 is an image showing color changes of assay samples having different concentrations of human neutrophil elastase (HNE), after approximately three minutes.
Figure 9 is an image showing color changes of assay samples having different concentrations of human neutrophil elastase (HNE), after approximately one hour.
Figure 10 is an image showing color changes of assay samples before adding trypsin, three minutes after adding trypsin, and one hour after adding trypsin.
Figure 11 is a graph showing turbidity for three different assay samples: urine without trypsin, urine with denatured trypsin, and urine with active trypsin.
Figure 12 shows an embodiment of a testing kit.
Figure 13 is an image showing the effect of pretreating an assay vessel with a siliconizing agent.
Figure 14 shows an embodiment E1 of a testing kit.
Figure 15 shows an embodiment E1 of a testing kit.
Figure 16 shows an embodiment E1 of a testing kit.
Figure 17 shows an embodiment E2 of a testing kit.
Figure 18 shows an embodiment E2 of a testing kit.
Figure 19 shows an embodiment E3 of a testing kit.
Figure 20 shows an embodiment E3 of a testing kit.
Figure 21 shows an embodiment E3 of a testing kit.
Figures 22A, 22B, and 22C show an embodiment E4 of a testing kit.
Figures 23A, 23B, and 23C show an embodiment E5 of a testing kit.
Figure 24 shows an embodiment E6 of a testing kit.
Figure 25 shows an embodiment of design characteristc CA of a testing kit.

### DETAILED DESCRIPTION OF THE INVENTION

The invention is defined in the appended claims, and illustrated by the following, non-limiting, embodiments.

In one embodiment, the invention includes a method of detecting protease activity in a sample, comprising contacting the sample with a composition comprising:
a dispersion of gold nanoparticles, whose surface is coated with a gelatin; and
CaCl₂;
wherein a color change of the composition, a turbidity change of the composition, or both, upon the contacting of the sample with the composition, indicates the presence of protease activity in the sample.

In an embodiment of the method, the gelatin coating the surface of the gold nanoparticles is an unmodified gelatin.

In an embodiment of the method, the composition does not comprise a dispersing agent.

In an embodiment of the method, the protease activity of the sample is protease activity of a matrix metalloprotease.

In an embodiment of the method, the color change indicates the presence of protease activity in the sample.

In an embodiment of the method, the turbidity change indicates the presence of protease activity in the sample.

In an embodiment of the method, both the color change and the turbidity change indicate the presence of protease activity in the sample.

In an embodiment of the method, the color change is a red to grey color change.

In an embodiment of the method, the color change, the turbidity change, or both, occur within one hour of the contacting of the sample with the composition.

In an embodiment of the method, the color change, the turbidity change, or both, occur within three minutes of the contacting of the sample with the composition.

In an embodiment of the method, the protease activity of the sample is protease activity of a human neutrophil elastase.

In an embodiment of the method, the method does not detect any enzymatically-inactive proteases in the sample.

In an embodiment of the method, the sample is fluid from a human wound.

In an embodiment of the method, the sample is fluid from a human wound, and the protease activity of the sample is protease activity of one or more bacterial proteases.

In an embodiment of the method, the sample is contacted with the composition in a container that has been surface treated to repel adsorption of the dispersion of gold nanoparticles whose surface is coated with the gelatin.

The method of the invention further comprises, prior to the contacting of the sample with the composition, preparing the composition by:
reducing tetrachloroauric acid with iso-ascorbic acid to obtain a dispersion of the gold nanoparticles in which the gold nanoparticles are uncapped;
coating the gold nanoparticles in the dispersion of gold nanoparticles with the gelatin, to obtain a dispersion of coated gold nanoparticles; and then
adding the CaCl₂ to the dispersion of coated gold nanoparticles.

In this embodiment, the reducing of tetrachloroauric acid with iso-ascorbic acid is preferably performed at room temperature.

In another embodiment, the invention includes a kit, comprising:
a vial containing a composition comprising CaCl₂ and a dispersion of gold nanoparticles whose surface is coated with a gelatin;
a cap configured for attachment to the vial, comprising a rubber septum; and
a sampling device configured for collecting a fluid from a wound.

The present invention describes a rapid, convenient, and reproducible method for the synthesis of stable gold sols by reducing tetrachloroauric acid with iso-ascorbic acid at room temperature (approximately 20°C). The resulting gold nanoparticles are quite uniform and their size can be adjusted by controlling the concentration and composition of Au(III) complexes. Since the preparation process does not require the addition of a dispersing agent such as dextrins and gums, or other polymers, the surface of the nanoparticles is amenable to a wide range of modifications useful for applications in medicine, biology, and catalysis.

The present invention describes a very simple and rapid precipitation process that uses iso-ascorbic acid (i-asc) as reductant and yields in several seconds at ambient temperature electrostatically stabilized gold sols in the absence of dispersing agents.

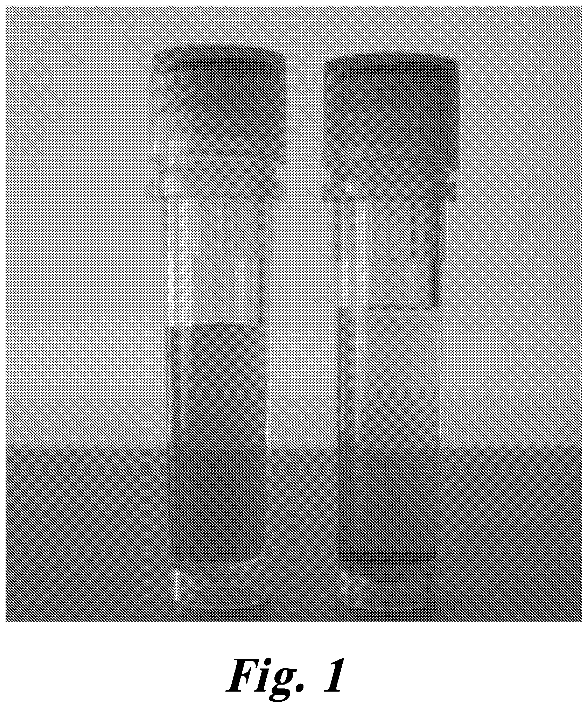

This section describes studies of the novel properties of titrated gelatin-coated gold nanoparticles which make up an important element for the assay for wound-associated proteases. Gelatin is derived from collagen, which is the most abundant of connective tissue proteins that make up the dermal layer. Pure melted gelatin chemisorbs onto the surface of metallic colloid nanoparticles as a result of hydrophobic interactions. In so doing, gelatin "protects" any negatively charged colloidal nanoparticles from the effects of charge reversal and electrolytic flocculation by shielding its surface charges. Thus, a concentration of electrolyte that will flocculate a quantity of bare gold colloid producing a red-to-blue-to-grey color change will not affect the same quantity of colloidal gold chemisorbed with protecting amounts of gelatin; under these conditions the gelatin-coated gold nanoparticle suspension remains red to the effect of a potentially flocculating amount of electrolyte. This phenomenon is shown in Fig. 1. The left-side tube in Fig. 1 contained gelatin-coated nanoparticles before protease hydrolysis, and the liquid was red in color (45 NTU). In contrast, the right-side tube contained gelatin-coated nanoparticles, but the gelatin-coated nanoparticles were exposed to protease hydrolysis, resulting in a liquid having a grey color (80 NTU) and with black flocculent.

According to one theory, this protection is achieved by loops forming on the surface of the nanospheres of gold in a "VW" pattern, where the gelatin is adsorbed to the surface of the colloid at hydrophobic areas represented by the points and ends of the "V" and "W", while the hydrophilic areas are unattached. Gelatin is more effective in protecting gold nanoparticles from flocculation on a molar basis than any other natural substance examined. The concentration of gelatin (100 kDa average size) needed to accomplish this protection is quite low: in one embodiment, just 16.6 picomol of pig skin gelatin will prevent the flocculation of commercially prepared 20 microgram sample of gold nanoparticles, 3.2 nanometer +/- 0.2 nm in a milliliter due to 0.33 mM CaCl₂.

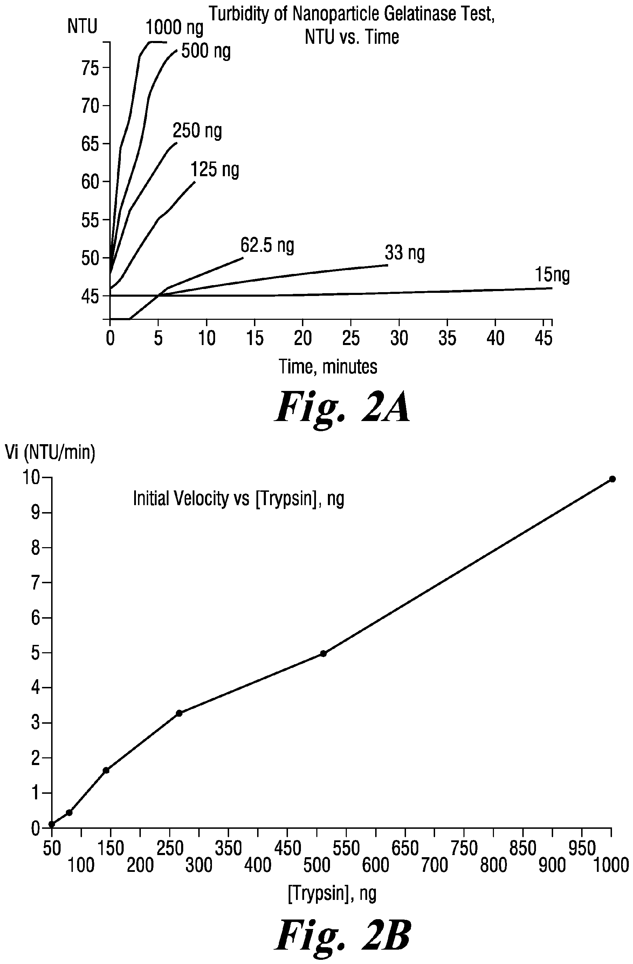

Proteases hydrolyze the gelatin on the surface of the nanoparticles, resulting in the loss of gelatin's integral structure and this abolishes its protection against charge reversal by the electrolyte. Once gelatin is hydrolyzed into polypeptides enzymatically, electrolytes like CaCl₂ act on the partially unprotected negatively-charged colloid nanoparticles and flocculate it in proportion to the extent of enzyme hydrolysis. As with any catalysis, the products formed in any time period are dependent upon the rate of gelatin hydrolysis. The flocculation of the suspended nanoparticles by the CaCl₂ as the result of the protease breakdown of gelatin in the liquid test can be followed in time by using an inexpensive nephelometer (*e*.*g*., a Hach 2100A or a portable instant read turbidimeter, a Lutron Model TU-2016), which measures the turbidity of the resulting flocculation of the gold nanoparticles with the varying protease amounts. Because the color change is a result of a localized surface plasmon resonance change and not a colorimetric change, the measurement is best suited for a turbidimetric rather than a spectrophotometric analysis. In one embodiment, a standard curve shown in Figs. 2A and 2B illustrates that the rate of turbidity change is linear from 33 to 1000 nanograms of purified bovine pancreas trypsin (EC 3.4.21.4), a serine gelatinase, in an incubation of 2 ml of gelatin-coated nanoparticles at room temperature. Flocculation of the gelatin-coated gold nanoparticles was also observed with other proteases as well, namely the cysteine protease bromelain (EC 3.4.22.33) from pineapple and actinidin (EC 3.4.22.14) from kiwi (data not shown).

Calcium ions act as the flocculent, and are a preferred flocculent because they are a required cofactor for matrix metalloproteases (MMPs). In MMPs, the polypeptide chain folds of the catalytic domains are essentially superimposable. The chain consists of a 5-stranded β-pleated sheet, three α-helices, and connective loops. This proteinase domain contains one catalytic zinc, one structural zinc, and, generally, three calcium ions. The substrate-binding cleft is formed by strand IV, helix B, and the extended loop region after helix B. Three histidines coordinate the active-site zinc. The loop region contains the conserved "Met-turn," a base to support the structure around the catalytic zinc. The fourth ligand of the catalytic zinc is a water molecule. The glutamic acid adjacent to the first histidine is essential for catalysis.

Preferably, the inventive assay uses an unmodified gelatin as the coating substrate for the gelatinases. In an embodiment, this unmodified gelatin is porcine. Traditional protease assays use a modified or synthesized form of gelatin or a gelatin-derivative that has a reporter group that is released upon cleavage and then measured, preferably instrumentally. An example of this is Mca- Lys-Pro-Leu-Gly-Leu-Dpa-Ala-Arg-NH₂, which has a fluorescent reporter group, Mca, which is released upon hydrolysis by gelatinases. Such a reporter group is not necessary in the present invention, since detection is achieved through flocculation of gold nanoparticles after their gelatin coating has been at least partially digested by a gelatinase. When using an unmodified gelatin as the coating substrate in the present invention, dipeptidases and exopeptidases do not affect the assay.

### Preparation of Colloidal Gold

The simultaneous mixing of tetrachloroauric acid and iso-ascorbic acid solutions results in the rapid formation of stable gold sols as indicated by the appearance of the typical red color of the final dispersion. The overall reduction reaction responsible for the formation of the dispersed gold is given by:

2HAuCl₄ + 3C₆H₈O₆ → 2Au⁰ + 3C₆H₆O₆ + 8HCl.

The use of iso-ascorbic acid instead of sodium citrate is advantageous in that using iso-ascorbic acid results in uncapped colloidal gold, whereas using sodium citrate results in citrate-capped colloidal gold.

In an exemplary embodiment, a 1% solution of tetrachloroauric acid was prepared by adding 1 gram of chloroaurate (tetrachloroauric acid, from Salt Lake Chemicals) to 100 ml water. To prepare the working gold sol solution, 0.88 ml of the 1% solution of tetrachloroauric acid was added to 100 ml distilled water. The pH of this was adjusted to pH 5.5 by adding 40 microliters of IN NaOH to it. Stock iso-ascorbic acid (i-asc, from Penta Chemicals) was prepared by dissolving 0.09 g in 50 ml distilled water and a working solution was prepared from the stock by adding 6.6 ml of stock to 93.4 ml of distilled water. This working solution was prepared freshly every time. Each of the 100 ml preparations of i-asc and chloroauric acid were mixed rapidly together in a larger mixing bowl and swirled gently until 200 ml of a wine red colored product of gold sol was produced. This mixing process was repeated four more times until 1000 ml of bare gold sol was produced. This preparation was 44 ppm gold sol of 40-50 nm approximate particle size.

### Preparation of Gelatin-Protected Gold Sol

The pH of the bare gold sol produced above was adjusted to a pH of 5.9 with 0.6 ml of IN NaOH (15 drops of 40 µl each) per 1000 ml of bare gold sol. Then the critical electrolyte concentration (CEC) of the bare gold sol was found with respect to 10 mM CaCl₂. To 50 ml of bare gold sol was added 2 ml of 10 mM CaCl₂. An immediate black precipitate occurred. The CaCl₂ stock solution was then sterile filtered it by passing it through a 0.22 micron filter.

Gelatin solution was made by first dissolving 70 mg pig skin type A gelatin in 50 ml distilled water. First Blooming for the gelatin for 10 minutes at room temperature in the distilled water, the mixture was then melted at 80°C. The pH then was adjusted to 10 with 1 ml IN NaOH. The solution was filter sterilized by passing it through a 0.22 micron nitrocellulose filter.

To prepare gelatin-protected gold sol (CPC, critical protection concentration): To 50 ml
of bare gold sol was added two drops (80 µl) of the above gelatin solution, with pouring rapidly 5 times from one 200 ml plastic vessel to another.

Two ml of 10 mM of the sterile filtered CaCl₂ solution was then added to this gelatin-protected gold sol. No precipitation occurred, unlike what occurred in the bare sol. Thus 2.25 µg gelatin/ml gold sol served to protect it from Ca⁺⁺ flocculation.

### Determination of Detection Limit for Trypsin with the Homogeneous Protease Test

Next, the detection limit of bovine pancreatic trypsin, a serine protease, was determined in a homogeneous (one step) assay for gelatinases (general proteases). Trypsin is a protease specific for arginine and lysine residues. Gelatin contains some 30+ of these residues/100,000 daltons. Trypsin thus hydrolyzes gelatin into 30+ parts, and is therefore considered a "gelatinase" protease.

Homogeneous Protease Test was prepared from a formula that consisted of 44 nanometer colloidal gold, pig skin gelatin and calcium chloride. It was contained in a 250 ml polyester terephthalate (PET) container that had been siliconized by adding a 1:10 dilution of Rain-X, decanting it and finally rinsing with distilled water. The Homogeneous Protease Test solution was treated with Triton X-100 at a concentration of 1:10,000 in order to prevent nonspecific adsorption binding (NSB) onto the polymeric surfaces. Prior to coating with gelatin, the gelatin solution, 1.0 mg/ml, and the CaCl₂ solution, 10 mM, were subjected to sterile filtration using a 0.22 micron filter. Ten ml of Homogeneous Protease Test was chosen to order to be read by a portable instant read turbidimeter, a Lutron Model TU-2016 which accepts and reads this quantity.

An aliquot of stock trypsin, 2 mg/ml, was thawed and diluted 1:5, 1:10, 1:20, 1:40, 1:80, and so on. Forty microliters of a given dilution was added to the 10 ml of Homogeneous Protease Test into a plastic 35ml container which was capless. Concentrations of trypsin added per ten ml of Homogeneous Protease Test were 10000, 5000, 2500, 1250, 650, or 313 ng of trypsin-protease. After mixing, the results were observed over a period of ten minutes and a period of one hour. The resulting color change was filmed by camera and assessed for flocculation by turbidimetry, with the result being an increase in turbidimetry value as an increase in flocculation of the colloidal gold, as shown in the following Table 1:

**Table 1**

| [Trypsin], ng | Turbidimeter Reading, NTU | Color |
|---|---|---|
| 10,000 | 577 | Black |
| 5000 | 618 | Blue |
| 2500 | 430 | Blue |
| 1250 | 100 | Purple |
| 625 | 32 | Red |
| 313 | 27 | Red |

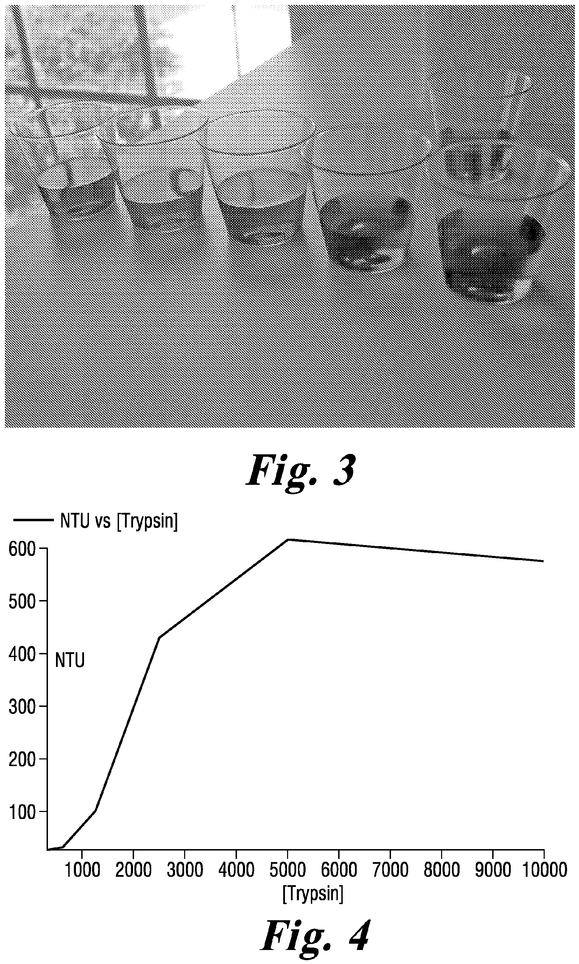

These results are also shown in Fig. 3, where in the front row, from left to right, are the samples of 10,000, 5000, 2500, 1250, and 625 ng trypsin, respectively, and in the back row is a negative control sample with no trypsin, after approximately one hour duration total incubation. The results are shown graphically in Fig. 4, with trypsin concentration (in ng per 10 ml) plotted on the abscissa, and the turbidimeter reading (in NTU) plotted on the ordinate. Based on these experiments, the detection lower limit for trypsin of the homogeneous protease test was determined to be 1250 ng in 1 hour for 10 ml of the testing solution.

### Detection Limit of Trypsin vs. Volume

With 10 ml samples of the homogeneous protease test solution described above, a graded colored response to protease was observed. Thus, black>blue>purple>red was observed for high to low trypsin additions. Because the enzyme catalyzes the substrate gelatin at different rates, the detection limit of the homogeneous protease test solution should vary with its volume. To test this hypothesis 0.5 and 0.25 ml volumes of the homogeneous protease test solution and trypsin, 10 µl, was added and the appearance of a color change or not indicated the detection limit at room temperature.

For 0.5 ml samples of the homogeneous protease test, the following results were obtained:

**Table 2**

| [Trypsin] | 20 µg | 2 µg | 200 ng | 20 ng | 0 (control) |
|---|---|---|---|---|---|
| Detection | + | + | + | - | - |

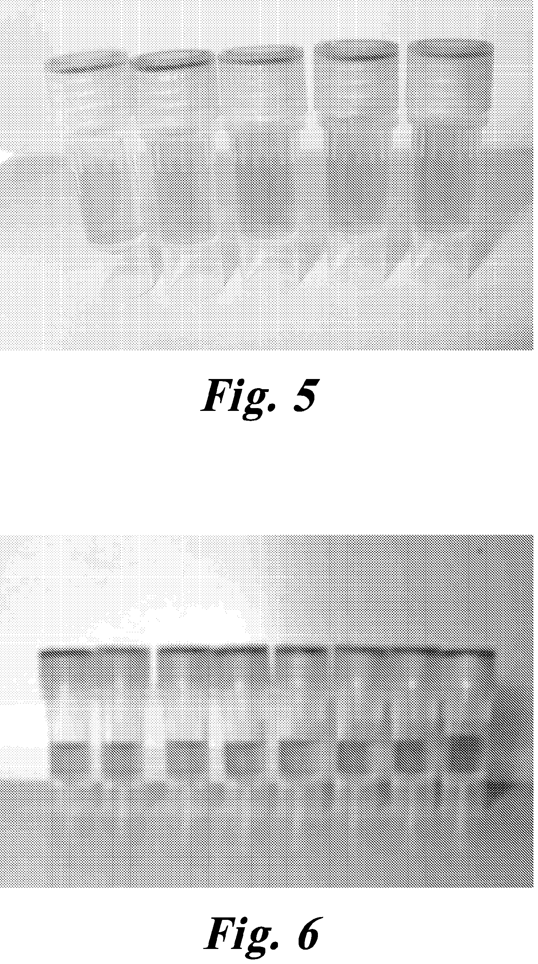

These samples are also shown in Fig. 5, where the samples are arranged from left to right as in the table above.

For 0.25 ml samples of the homogeneous protease test, the following results were obtained:

**Table 3**

| [Trypsin] | 20 µg | 2 µg | 200 ng | 20 ng | 2 ng | 0.2 ng | 0.02 ng | 0 (control) |
|---|---|---|---|---|---|---|---|---|
| Detection | + | + | + | + | - | - | - | - |

These samples are also shown in Fig. 6, where the samples are arranged from left to right as in the table above.

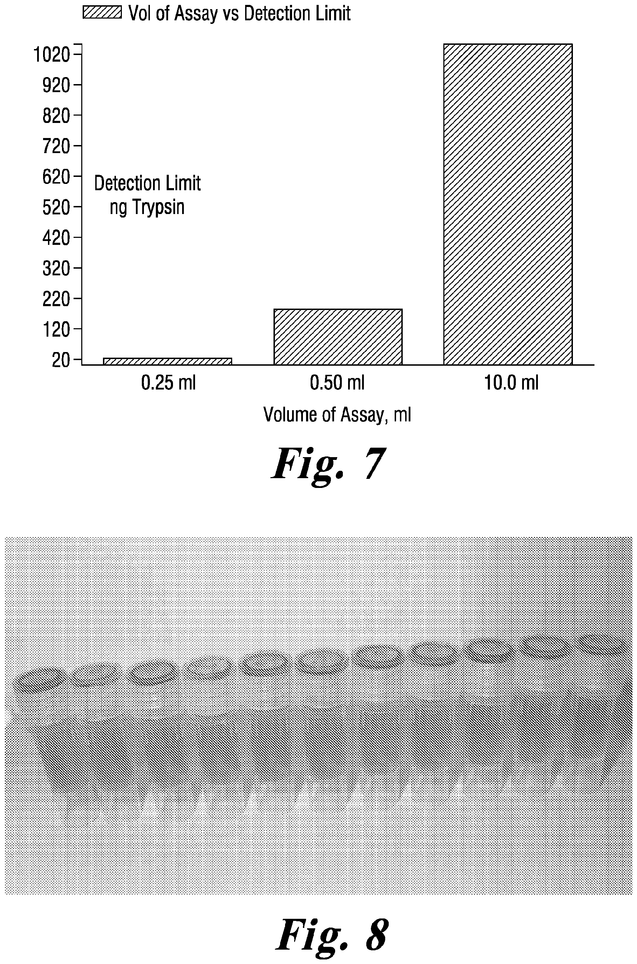

This dependence of detection limit based on sample volume is shown graphically in Fig. 7. The detection limit varies with the volume of homogeneous protease test: 1250 ng, 200 ng and 20 ng for 10 ml, 0.5 ml and 0.25 of the homogeneous protease test, respectively.

It was observed that the preparation as described above for the homogeneous protease test was stable for at least two months at room temperature.

### Detection of Human Neutrophil Elastase

Human neutrophil elastase (HNE) is a non-metalloprotease, and is a human protease associated with wounds. Using the homogeneous protease test of the present invention, active HNE was detectable for the first time.

The inventive homogeneous protease assay was used to detect HNE. HNE, also known as leukocyte elastase, is a serine non-metalloprotease which, along with the matrix metalloproteases, particularly MMP-2 and MMP-9 (also known as Gelatinases A and B, respectively) are known to be elevated in chronic wounds.

Ten microliters of HNE (Innovative Research, 1.3 mg/ml, lot 44726) were diluted neat (undiluted), 1:10, then 1:100, and 1:1000 in distilled water. A water negative control, 10 µl, was also included. To each 0.5 ml aliquot of homogeneous (one-step) colorimetric protease assay, was added: 10, 20, 30 ... 100 µl of the 1:1000 dilution of HNE, representing: 13, 26 ...130 ng of the protease HNE. The reaction, if any, was observed as a color change from red color to grey within one minute, and a further reaction, if any, was observed as a flocculation in which the grey suspension settled out of solution in time, leaving a clear supernatant. The results were photographed at approximately 3 minutes and after approximately 1 hour duration.

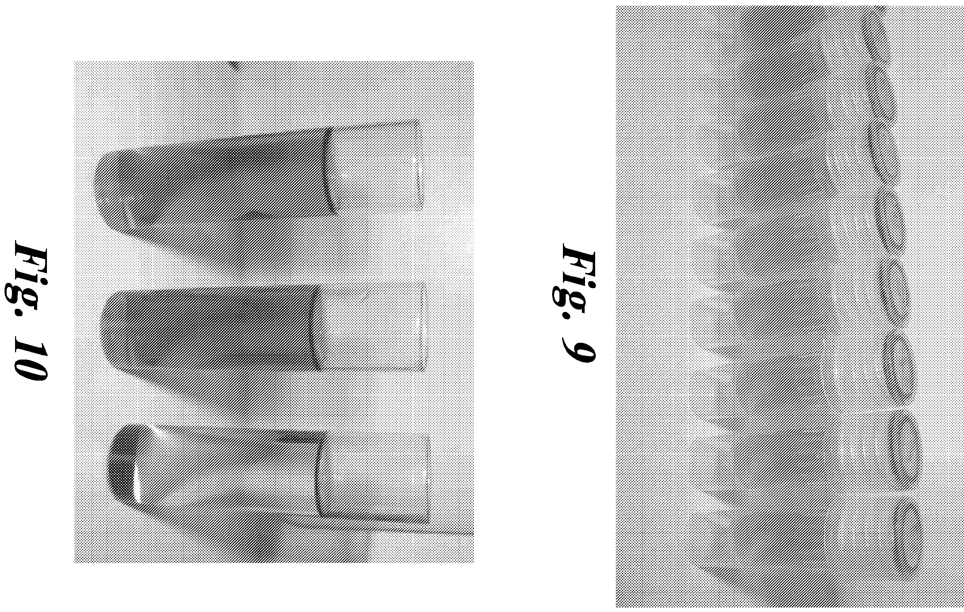

Figs. 8 and 9 show the results of the HNE assay. From left to right in Figure 8 is shown the samples of 100, 90, 80, 70, 60, 50, 40, 30, 20, 10, and 0 µl of 1:1000 dilution of 1.3 mg/ml of HNE and 10.0 µl of water (control), representing 130, 117, 104, 91, 78, 65, 52, 39, 26, 13, 0.0 ng HNE, respectively, after approximately 3 minutes. Fig. 9 shows the same 80, 70, 60, 50, 40, 30, 20, 10, and 0 µl samples of Fig. 8, but after approximately one hour. From Figs. 8 and 9, the detection limit for HNE was determined to be 26 ng per 0.5 ml of homogeneous (one step) colorimetric protease assay.

### Detection of Bacterial Proteases

It is well known that some bacteria that infect wounds, for example *S. aureus* and P. *aeruginosa,* secrete gelatinases. To test this, wound fluid isolated from bandages were collected and assayed for proteases and for bacteria. Twenty clinical samples were assayed for proteases using a fluorescent FRET-based assay that detects bacterial proteases (BPA), bacterial colony counts using known methods for growing and enumerating them, and the inventive colorimetric assay of the present application. Ten microliters of clinical sample was added to one milliliter of the assay reagents. The results are summarized in the following Table 4:

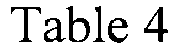

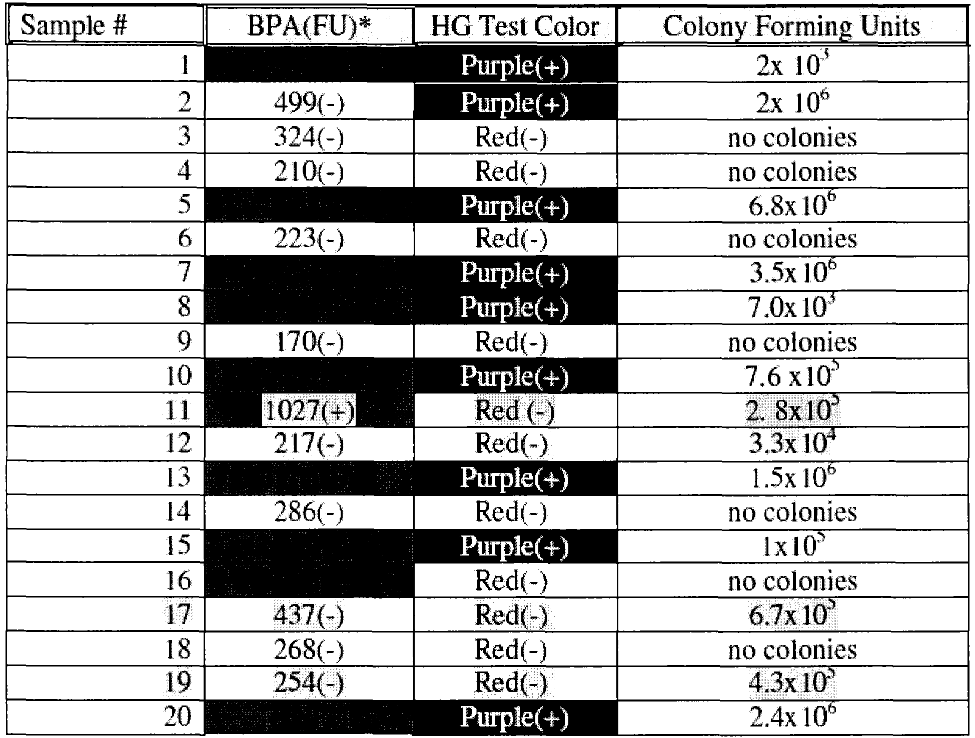

### The Assay Measures Active Enzyme, Not Denatured Enzyme

An image of the one-step gelatinase test reacting with bovine pancreas trypsin (EC 3.4.21.4), a serine endoprotease, in a turbidimeter tube is shown in Fig. 10. The color changed from red (tube 1, left) to gray (tube 2, middle) within three minutes, then became clear (tube 3, right) after an hour as aggregated flocs settled by gravity.

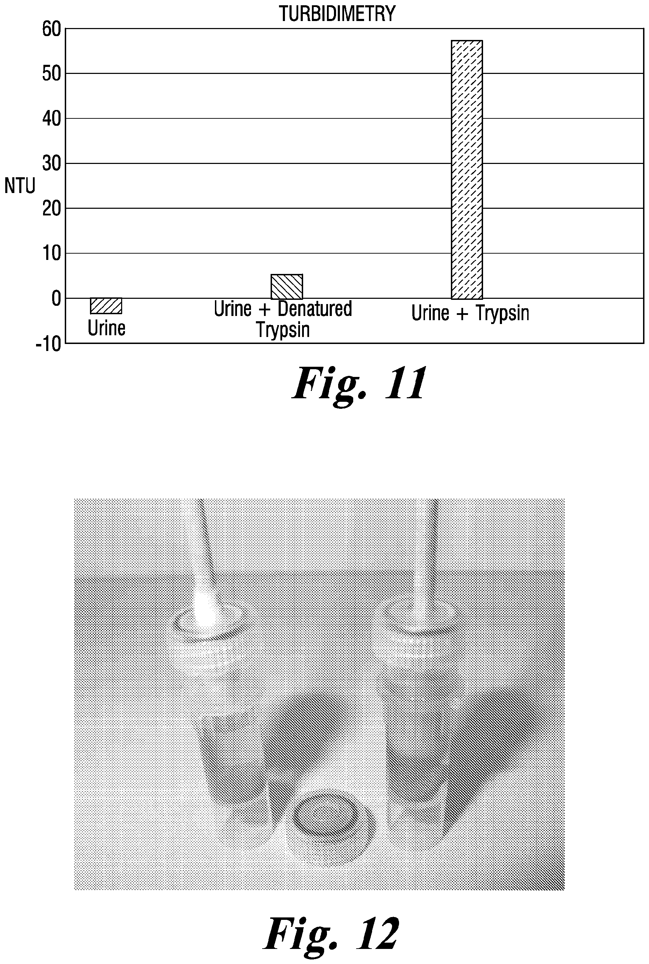

Fig. 11 shows the corresponding change in turbidity due to the addition of the active trypsin. The left bar indicates the turbidity prior to adding active trypsin, and the right bar indicates the turbidity three minutes after adding the active trypsin. Importantly, the addition of inactive, heat denatured trypsin had no effect on the color (photo not shown) and only a slight effect on the measured turbidity, as shown in the center bar of Fig. 11. This experiment shows that the assay only measures active enzyme, not denatured enzyme. This characteristic differs from immunohistochemical assays, which do not necessarily distinguish between active and inactive enzymes.

### Kit for Performing the Assay

Embodiments of the invention include diagnostic kits for performing the inventive assay, for example testing for the presence of proteases in fluid from a wound. Such a kit allows for a homogeneous, one-step sampling and assessment of elevated proteases in various biofluids such as wound exudate.

Such a kit may include 1) a vial of a mixture of exactly-titrated colloidal gold-gelatin-CaCl₂, 2) a cap that has a rubber septum which is fluid- or water-tight for storage, and 3) a sampling device for wound fluid, preferably a swab-type device for picking up and distributing the wound fluid into the vial of said homogeneous protease test fluid. In embodiments, the vial is made of glass or plastic. The swab is preferably of a nature that does not absorb the protease irreversibly, as, for example, polypropylene or flocked nylon. In an embodiment, the swab includes a non-cotton or non-cellulose material. An embodiment of a suitable testing kit is depicted in Fig. 12, which shows two plastic vials before (left side) and after (right side) reaction, and a cap containing a releasable rubber septum.

In an embodiment, once the wound fluid sample is obtained by the swab, the swab is inserted into the vial by releasing the rubber septum. Afterwards, the swab contents are released into the vial contents by the friction of the open circle on the cap after release of the septum and the swab; this is depicted in the vial on the left in Fig. 12.

Once the contents of the swab are released into the vial, the proteases in the wound sample react with the protease-sensing solution, changing its color. This is depicted in the vial on the right in Fig. 12.

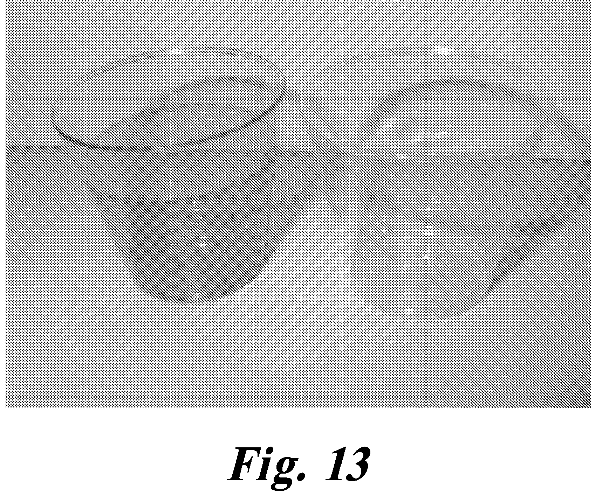

In a preferred embodiment, the inside surface of the vial is treated, for example by siliconizing it. This may be beneficial when the three components of colloidal gold, gelatin, and Ca⁺⁺ ions combine to form a sticky fluid. This effect can be seen in Fig. 13, which depicts two polyethylene vessels, the one on the left untreated and the one on the right treated with a siliconizing agent, and each holding colloidal gold-gelatin-Ca⁺⁺ mixture. As can be seen in the left vessel, the surface adsorbs the colloidal gold-gelatin-Ca⁺⁺ mixture with the appearance of a visual color, leading to an eventual and gradual loss of signal; while the treated vessel on the right side does not. Embodiments of the surface treatment may include Siliclad, octadectyl silane, a water soluble siliconizing agent, RAIN-X, a commercially available water soluble siliconizing agent or any manner of surface treatment that repels the adsorption of small amounts of gelatin that may adsorb to the surfaces of the vial.

The vial contains the assay reagents in one or more reservoirs, to contain the reagents either pre-introduction of biofluid or both pre-and post-introduction, *e.g*., a single reservoir both pre-and post-introduction of biofluid. The sampling device may or may not be integrated with the vial, *e.g.,* the sampling device might also contain an integrated reservoir(s). In an embodiment, a pre-introduction reservoir serves to contain and preserve the assay reagents until they are mixed with the collected biofluid sample. A post-introduction reservoir may serve as the mixing reservoir, containing the assay reagents and a means to introduce the biofluid sample. The post-introduction reservoir may remain contained or re-seal after sample introduction, possibly also containing a swab tip or other collection apparatus part or whole. In an embodiment, the post-introduction reservoir is transparent and marked with a color scale to visually observe and easily match the color of the assay to the scale to assign a numeric and/or binary high/low value of protease levels.

Six exemplary embodiments of kit designs are described below (E1, E2, E3, E4, E5, and E6) with four additional exemplary design characteristics (CA, CB, CC, and CD), whereby one or more of the four design characteristics could apply any of the six kit designs.

The kit for transport and processing of the assay reagents, with any biofluid, e.g. human wound exudate or fluid which contains a protease or an enzyme which hydrolyzes gelatin, comprises three primary components: the first is the collection apparatus or sampling device; the second is the reservoir that stores the assay reagents until ready for use; the third is the reagent reservoir where the assay reagents and the biofluid sample are combined.

These three components may be separate forms or may be integrated to serve multiple purposes. For example, the storage reservoir for the assay pre-mixing might be the same reservoir in which the biofluid and the assay reagents are combined after sampling and transport to the kit.

In an embodiment, the collection apparatus or sampling device is a swab or similar device which is used to sample the biofluid. The swab-type device is preferably not cellulose-based or of cotton-like material, as that has been shown in our studies to adsorb protease enzymes irreversibly.

The second component, the reservoir or vessel that holds the pre-sample assay reagents, whether made of plastic or glass, is preferably surface treated, *e.g.*, siliconized, to prevent adsorption of the gelatin-colloidal metal.

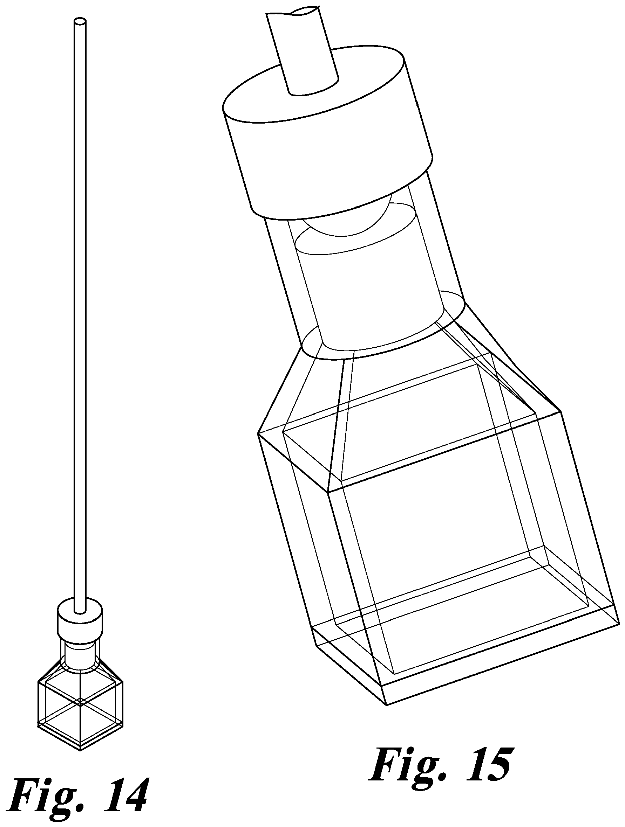

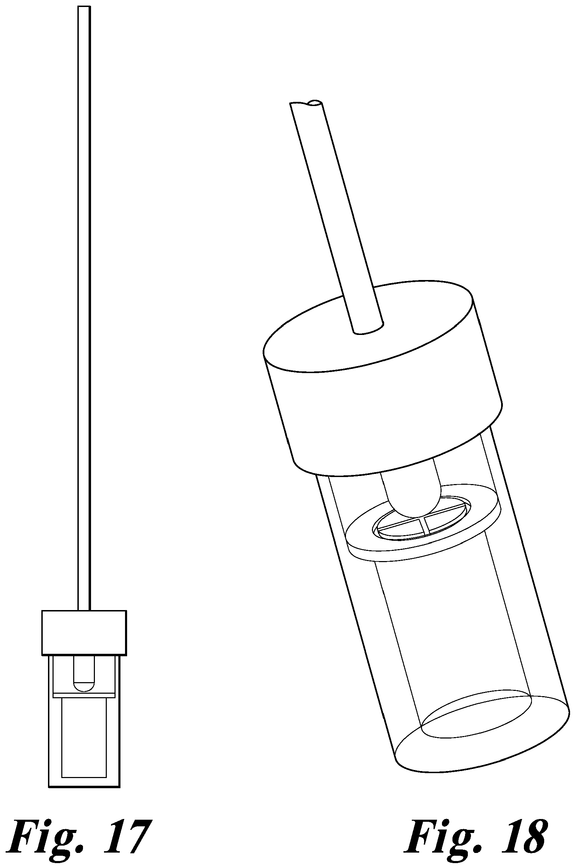

Embodiments E1 (Figs. 14-16) and E2 (Figs. 17 and 18) share the following characteristics:
1. A swab for sampling (of various dimensions and properties, shown as a standard 6" stem swab)
2. One or two caps: capA to seal the reservoir, and capB to seal the swab into the device. The device may have one cap such that the cap that seals the reservoir can be removed, impaled by the swab shaft, and then used to also seal the post-sample swab to the reservoir. The device might have two caps, capA sealing the reservoir until sample is collected and ready to introduce into the assay reagents. CapB is either a separate item in the kit for the device or is already impaled by the swab as a single component.
3. A reservoir that contains the assay reagents and that may have a redundant seal internal to the reservoir.

Embodiment E1 is shown in Figs. 14-16. Fig. 14 shows one version of the starting kit. One cap, impaled by the swab, seals the swab to the reservoir. The assay reagents are separated from the swab holding chamber, containing air and the tip of the swab, by a stopper, made from various materials such as cork, cotton, or other common fluid stopper material, that does not permit the flow of fluid across the barrier and that preserves the assay reagents until ready for use. Fig. 15 shows a close-up view of part of Fig. 14.

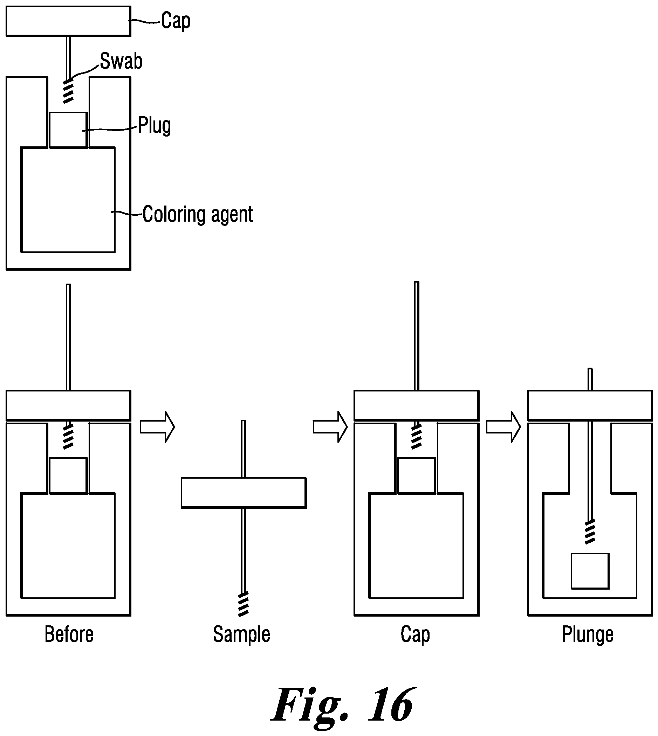

To use the kit in this embodiment, the cap is unscrewed to release the cap/swab combo from the reservoir (see Fig. 16). The cap can be slid or pushed manually along the swab stem to provide greater freedom to swab biofluid, *i.e.,* create longer post-cap stem so that the swab can be, for example, rolled around and otherwise manipulated in a wound to saturate the tip without interference from the cap. When the swab tip is saturated, the cap is manually slid back down to within 1 - 20 mm of the start of the swab fibers. Then, the cap/swab component is re-screwed onto the reservoir such that the tip does not extend enough to contact the stopper or if touching, not enough to fully disengage the stopper until the cap screw threads are fully tightened. See Fig. 16.

Once the threads are fully tightened, ensuring a watertight seal, the swab stem is pushed down into the reservoir, pushing the stopper out of place and into the assay reagents. The swab is pushed all the way down until the tip extends as far it can, contacting the narrow most contour or the bottom face of the inner reservoir walls such that it cannot extend further.

A variation of embodiment E1 includes two caps, capA sealing the reservoir with or without the redundant inner stopper seal, plus capB which is already impaled by the swab such that the swab/capB combo is ready for biofluid sampling. When the swab with sampled biofluid is ready to be inserted into the reservoir, capA is removed and discarded, and the process then continues similar to the single cap version above.

Embodiment E2 is shown in Figs. 17 and 18. The primary variation from Embodiment E1 is the internal assay reagent barrier method. Fig. 17 shows one version of the starting kit. One cap, impaled by the swab, seals the swab to the reservoir. The assay reagents are separated from the swab holding chamber, containing air and the tip of the swab, by a barrier, made various rigid or flexible material such as plastics or rubbers, that do not permit the flow of fluid across the barrier and that break or give way to downward pressure applied to the swab stem. Fig. 18 shows a close-up view of part of Fig. 17.

The second variation from Embodiment E1 in Embodiment E2 is the shape of the inner and outer contours of the kit. Embodiment E2 has a cylindrical outer contour and a smaller cylindrical inner contour. The inner and outer can take any of various shapes, see design characteristic CA below.

After sampling biofluid, the cap is slid down to the swab end and the threads in the cap are tightened onto the reservoir threads. Then, the swab stem is pushed down into the reservoir, breaking or penetrating the barrier and introduced into the assay reagents. The swab is pushed all the way down until the tip extends as far it can, contacting the narrow most contour or the bottom face of the inner reservoir walls such that it cannot extend further.

A variation of Embodiment E2 includes two caps, capA sealing the reservoir with or without the redundant inner seal, plus capB which is already impaled by the swab such that the swab/capB combination is ready for biofluid sampling. When the swab with sampled biofluid is ready to be inserted into the reservoir, capA is removed and discarded, and the process then continues similarly to the single cap version above.

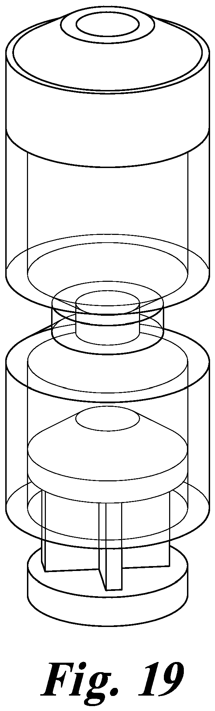

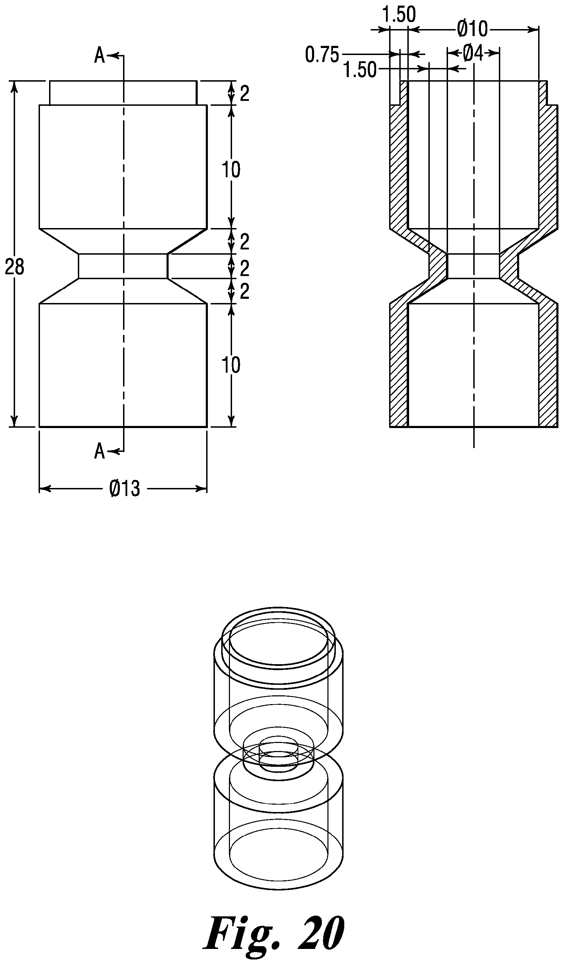

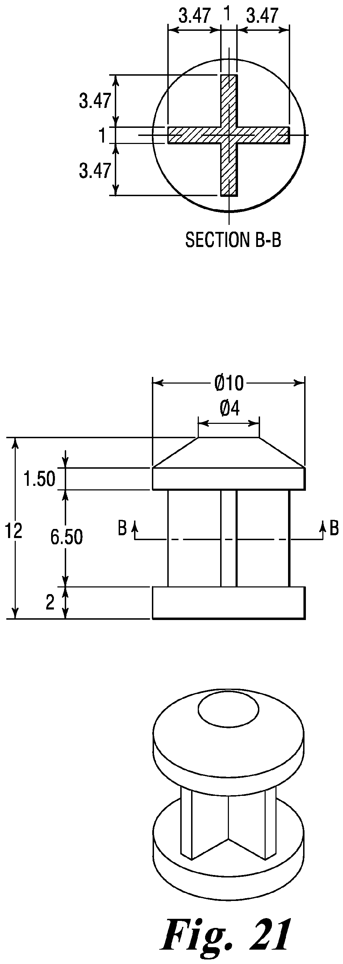

Embodiment E3 is shown in Figs. 19-21. This embodiment is conceptually akin to two needle-less syringes molded together at their tips. One "syringe" reservoir contains the assay reagents, in waiting for sample insertion into the other. It has a characteristic plunger with stopper head. There is a barrier material at the juncture of the two "syringes" such that the assay reagents are contained and rendered immobile in the waiting reservoir. The other half of the device has a cap with a membrane that is easily puncturable by a swab stem but that seals around the stem (water tight). Fig. 20 is a schematic version of Fig. 19, and Fig. 21 is an isolated view and schematic view of the actuator stem in Fig. 19.

Like in the E1 and E2 single cap embodiments, the cap is removed, impaled by the swab stem at the opposite end of the swab tip, and slid part way down the stem so as to not interfere with sampling. After sampling, the cap is slid down towards the swab fibers as described in E1 and E2 and re-sealed onto the sample receiving end of E3. Once sealed tight, the assay reagent reservoir plunger on the opposite end of E3 is plunged completely to force the stopper through the narrow channel connecting the two halves of E3, also forcing the entire volume of assay reagents from the holding side into the sampling side until the stopper is in its fully plunged position. The swab can then be extended until it impacts the bottom surface of the sampling side, entirely submerged in assay reagents.

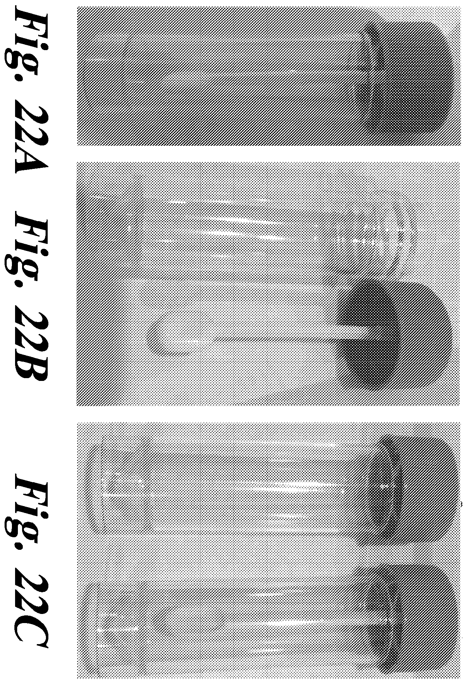

Embodiment E4 is shown in Figs. 22A, 22B and 22C. This embodiment has a single cap/swab combination design whereby the swab stem is between 30 - 100 mm in length above the upper end of the sampling fibers to the inner façade of the screw cap to allow more degrees of freedom to sample biofluid. The swab stem terminates in the cap such that swab and cap are a single piece, either manufactured as such or otherwise affixed to the inside surface of the cap, such as with a female anchor feature inside the cap that fits the swab stem tightly once inserted, so that the cap itself can be used as the manipulation handle to broadly sample biofluid. See Figs. 22A and 22B.

In one embodiment, shown in Fig. 22C, there are two vials/containers: one (VI) contains the assay reagents and a standard cap, *i.e.,* no swab/cap combo. The second vial (V2) may/may not be identical in shape and size to V1, but will have the following key differences: (1) the inner volume is empty (air), and (2) the cap is the cap/swab combo. A clinician would unscrew or otherwise remove the swab/cap combo from the protective vial V2, swab biofluid, remove the standard cap from V1, and replace and seal V1 with the sampled swab/cap combo from V2, thereby inserting the biofluid sample into the assay reagents in V1.

Also contemplated are variations that combine features of embodiments E1, E2, E3, and E4. For example, V1 of E4 might have a redundant inner seal described in E2 or E3.

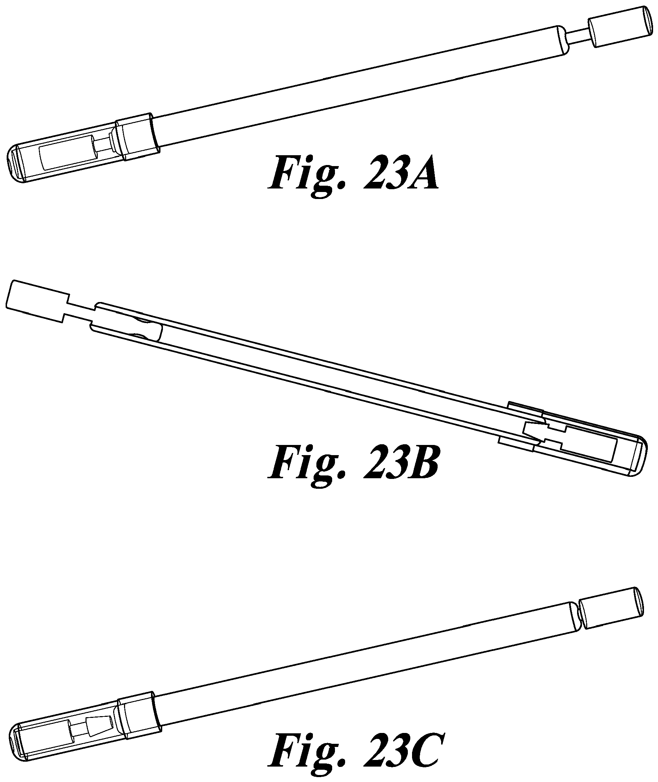

Embodiment E5 is shown in Figs. 23A, 23B and 23C. E5 is a device in appearance generally similar in shape to a ball point pen. The body of the device contains a longitudinal reservoir, either the hollowed chamber itself or a nested chamber or pouch made from a shape-shifting/flexible or rigid material, which holds the assay reagents, pre-mixing, which is enclosed in the smooth "pen" encasement that forms the handle by which to sample biofluid. The encasement is cylindrical in shape or prismatic or irregular to include groves or contours for easy handling and manipulation in sampling biofluid such as from a wound.

From one end of the device, a translucent cap is removed to expose a swab tip that extends between 1.0 - 5.0 cm from the end of the device. The clinician then samples biofluid, saturating the swab tip. The cap is replaced and locks in place, water-tight. The assay reagents are released in one of the following ways: (a) the cap is designed with internal features that serve as teeth or knives or levers, varying by the materials used in and the design of the internal reagent holding reservoir, such that the cap is locked and then turned 45 - 360 degrees, puncturing or opening or otherwise releasing the assay reagents from their holding reservoir into the translucent cap, (b) the device has a manual trigger, such as with ball point pen click lever, on the top end or on the side such as a switch lever, whereby the depression or sliding of the lever or button triggers release of the assay reagents into the waiting reservoir in the cap by action of puncturing or opening or otherwise releasing, such as if the swab attachment to the pen body is itself a cork or stopper for the assay reagent reservoir (as pictured), the assay reagents from their holding reservoir into the translucent cap. In an embodiment, the trigger of release of assay reagents allows the reagents to flow freely using gravity into the translucent cap, submerging the biofluid-saturated swab in assay reagents 2mm - 20mm above the highest point where the swab fibers are no longer protruding from the swab shaft.

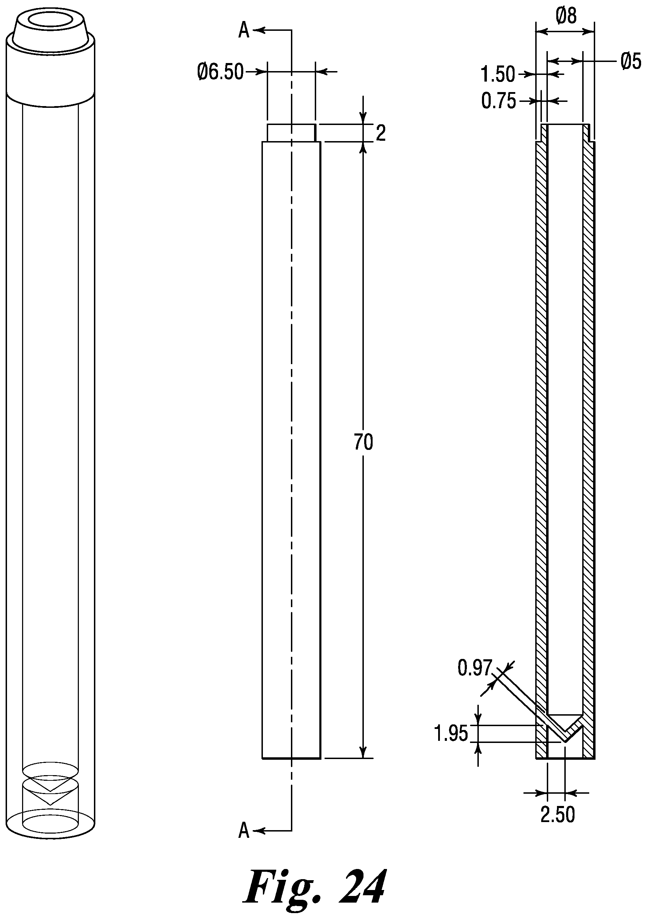

Embodiment E6 is shown in Fig. 24. This embodiment resembles a test tube, but with a flat bottom either solid or hollowed/ringed so that the tube stands on its own. The tube contains the assay reagents. A separate swab is used to sample biofluid, similar to the process in E3: the cap is removed, impaled by the swab (opposite end of the swab tip) prior to sampling, sampling, sliding the cap to within 1 - 10 mm of the highest swab bristles without touching them, screwing/tightening the cap/swab combo to the tube section of E6, then extending the swab until the tip impacts the bottommost surface of the tube.

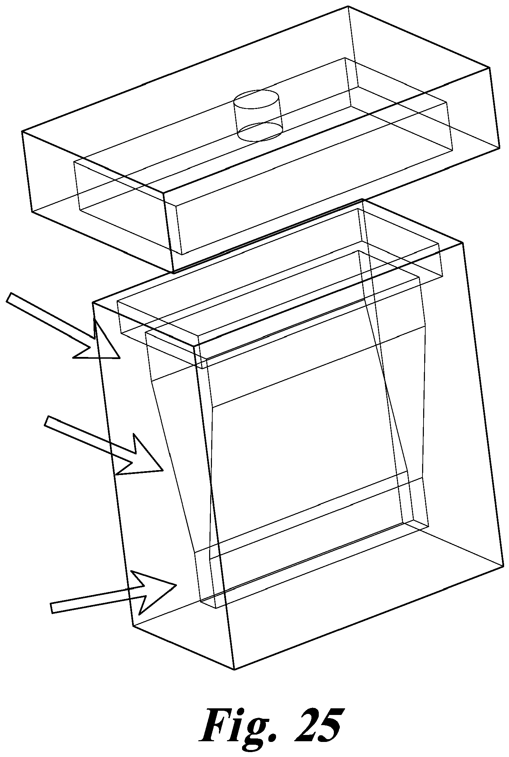

The following exemplary embodiments of design characteristics (CA, CB, CC, and CD), may be employed, alone or in any combination, to any of the six embodiments of kit designs E1, E2, E3, E4, E5 and E6 described above. These design characteristics are described with reference to Fig. 25.

Design characteristic CA relates to the inner and outer contours and shapes of the kit/device. For example, the outer shape of the assay reagent reservoir in Embodiments E1-E3 may be, in certain embodiments, rectangular, cylindrical, or prismatic, whereby the base is rectangular, circular, triangular, or some other flat shape to allow the reservoir to stand on a flat surface on its own.

In CA-1, the inner well mirrors the shape and contours of the outer well.

In CA-2, the inner well differs in contour from the outer surface to create inner well sections that allow longer path length viewing of the HPA. The contemplated shapes are rectangular, pyramidal, and/or square prisms, either singly employed or in combination such as an upside down triangular prism that opens in to a much narrower rectangular prism to create a narrow and long channel of assay reagents for enhanced color viewing.

The embodiment of design characteristic CA depicted in Fig. 25 shows a reservoir combining a rectangular prism shape (upper arrow) above a trapezoidal prism shape (middle arrow) above a smaller rectangular prism shape (lower arrow).

Design characteristic CB relates to a measurement scale on the kit/device. A label, either on the exterior surface or interior surface of the mixing or sampling reservoir can guide the clinician to a semi-quantitative assessment of protease levels. The scale may be one continuous segment, or may be discrete and separate segments, and may be rectangular, triangular or a variation (such as a triangular shape with one side cured vs. linear to help visually indicate the high end of the color spectrum vs. the low end). The color grades may be in 2 (binary high/low), 3, 4, 5, 6, 7, 8, 9, or 10 grade increments. The label may be affixed, etched, printed, or otherwise directly placed on the inner or outer surface so that the assay can be viewed immediately adjacent to the color grades that comprise the scale.

Design characteristic CC relates to a background of the kit/device. White or similarly reflective, non-interfering color or background, by any means of application, whether painted, adhered/glued, and whether internal or external, may be used on or around the device to both control the amount of light that enters the vial and to enhance the appearance of the assay color for more consistent color reading. One embodiment is a bright white label adhered to the backside of the vial that wraps around 40-80% of the vial to create optimal optics to observe assay color.

Design characteristic CD relates to magnification or other optical manipulation used in the kit/device. Secondary optics may be used in the kit/device, such as a Fresnel lens or a layer or lens that has the opposite effect (distancing vs. magnifying), affixed inside or outside the reservoir containing the assay reagents, to make the observation/reading of the color more consistent and/or easier. Such optics also may be incorporated into the structure or walls of the vial.

## Claims

1. A method of detecting protease activity in a sample, comprising contacting the sample with a composition comprising:
a dispersion of gold nanoparticles, whose surface is coated with a gelatin; and
CaCl₂;
wherein a color change of the composition, a turbidity change of the composition, or both, upon the contacting of the sample with the composition, indicates the presence of protease activity in the sample,
**characterized in that** the method further comprises, prior to the contacting of the sample with the composition, preparing the composition by:
reducing tetrachloroauric acid with iso-ascorbic acid to obtain a dispersion of the gold nanoparticles in which the gold nanoparticles are uncapped;
coating the gold nanoparticles in the dispersion of gold nanoparticles with the gelatin, to obtain a dispersion of coated gold nanoparticles; and then adding the CaCl₂ to the dispersion of coated gold nanoparticles.

2. The method of claim 1, wherein the gelatin coating the surface of the gold nanoparticles is an unmodified gelatin.

3. The method of claim 1, wherein the composition does not comprise a dispersing agent.

4. The method of claim 1, wherein the protease activity of the sample is protease activity of a matrix metalloprotease.

5. The method of claim 1, wherein the color change indicates the presence of protease activity in the sample, preferably wherein the color change is a red to grey color change.

6. The method of claim 1, wherein the turbidity change indicates the presence of protease activity in the sample.

7. The method of claim 1, wherein both the color change and the turbidity change indicate the presence of protease activity in the sample.

8. The method of claim 1, wherein (i) the color change, the turbidity change, or both, occur within one hour of the contacting of the sample with the composition; or:
wherein (ii) the color change, the turbidity change, or both, occur within three minutes of the contacting of the sample with the composition.

9. The method of claim 1, wherein the protease activity of the sample is protease activity of a human neutrophil elastase.

10. The method of claim 1, which does not detect any enzymatically-inactive proteases in the sample.

11. The method of claim 1, wherein the sample is fluid from a human wound, preferably wherein the protease activity of the sample is protease activity of one or more bacterial proteases.

12. The method of claim 1, wherein the sample is contacted with the composition in a container that has been surface treated to repel adsorption of the dispersion of gold nanoparticles whose surface is coated with the gelatin.

13. The method of claim 1, wherein the reducing of tetrachloroauric acid with iso-ascorbic acid is performed at room temperature.

14. A kit, comprising:
a vial containing a composition comprising CaCl₂ and a dispersion of gold nanoparticles whose surface is coated with a gelatin;
a cap configured for attachment to the vial, comprising a rubber septum; and
a sampling device configured for collecting a fluid from a wound.

## Patentansprüche

1. Verfahren zum Nachweis der Proteaseaktivität in einer Probe, welches ein In-Kontakt-Bringen der Probe mit einer Zusammensetzung umfasst, die aufweist:
eine Dispersion von Goldnanopartikeln, deren Oberfläche mit einer Gelatine beschichtet worden ist, und
ein CaCl₂,
wobei eine Farbänderung der Zusammensetzung, eine Trübungsänderung der Zusammensetzung oder beides bei dem In-Kontakt-Bringen der Probe mit der Zusammensetzung das Vorhandensein einer Proteaseaktivität in der Probe anzeigt, **dadurch gekennzeichnet, dass**
das Verfahren ferner vor dem In-Kontakt-Bringen der Probe mit der Zusammensetzung ein Herstellen der Zusammensetzung umfasst, durch:
ein Reduzieren von Tetrachlorgoldsäure mit einer Isoascorbinsäure, um eine Dispersion der Goldnanopartikel zu erhalten, in der Umhüllungen der Goldnanopartikel entfernt worden sind,
ein Beschichten der Goldnanopartikel in der Dispersion der Goldnanopartikeln mit der Gelatine, um eine Dispersion von beschichteten Goldnanopartikeln zu erhalten, und dann
ein Hinzufügern des CaCl₂ an die Dispersion der beschichteten Goldnanopartikel.

2. Verfahren nach Anspruch 1, wobei die Gelatine, die die Oberfläche der Goldnanopartikel beschichtet, eine nicht modifizierte Gelatine ist.

3. Verfahren nach Anspruch 1, wobei die Zusammensetzung kein Dispergiermittel enthält.

4. Verfahren nach Anspruch 1, wobei die Proteaseaktivität der Probe die Proteaseaktivität einer Matrixmetalloprotease ist.

5. Verfahren nach Anspruch 1, wobei die Farbänderung das Vorhandensein einer Proteaseaktivität in der Probe anzeigt, wobei die Farbänderung bevorzugt eine Farbänderung von rot nach grau ist.

6. Verfahren nach Anspruch 1, wobei die Trübungsänderung das Vorhandensein einer Proteaseaktivität in der Probe anzeigt.

7. Verfahren nach Anspruch 1, wobei sowohl die Farbänderung als auch die Trübungsänderung das Vorhandensein einer Proteaseaktivität in der Probe anzeigen.

8. Verfahren nach Anspruch 1, wobei (i) die Farbänderung, die Trübungsänderung oder beide innerhalb einer Stunde nach dem In-Kontakt-Bringen der Probe mit der Zusammensetzung auftreten, oder
wobei (ii) die Farbänderung, die Trübungsänderung oder beide innerhalb von drei Minuten nach dem In-Kontakt-Bringen der Probe mit der Zusammensetzung auftreten.

9. Verfahren nach Anspruch 1, wobei die Proteaseaktivität der Probe eine Proteaseaktivität einer menschlichen neutrophilen Elastase ist.

10. Verfahren nach Anspruch 1, das keine enzymatisch inaktiven Proteasen in der Probe nachweist.

11. Verfahren nach Anspruch 1, wobei die Probe eine Flüssigkeit aus einer menschlichen Wunde ist, wobei die Proteaseaktivität der Probe bevorzugt eine Proteaseaktivität einer oder mehrerer bakterieller Proteasen ist.

12. Verfahren nach Anspruch 1, wobei die Probe mit der Zusammensetzung in einem Behälter in Kontakt gebracht wird, der zur Abstoßung der Adsorption der Dispersion der Goldnanopartikel, deren Oberfläche mit der Gelatine beschichtet wurde, oberflächenbehandelt worden ist.

13. Verfahren nach Anspruch 1, wobei das Reduzieren der Tetrachlorgoldsäure mit einer Isoascorbinsäure bei Raumtemperatur durchgeführt wird.

14. Ausrüstung, die aufweist:
eine Phiole, die eine Zusammensetzung enthält, die ein CaCl₂ und eine Dispersion von Goldnanopartikeln aufweist, deren Oberfläche mit einer Gelatine beschichtet worden ist,
eine Kappe, die zum Anbringen an der Ampulle ausgestaltet ist und die ein Gummiseptum aufweist, und
eine Probenahmevorrichtung, die zum Sammeln einer Flüssigkeit aus einer Wunde ausgestaltet ist.

## Revendications

1. Procédé de détection d'activité protéase dans un échantillon, comprenant la mise en contact de l'échantillon avec une composition comprenant :
une dispersion de nanoparticules d'or, dont la surface est enrobée d'une gélatine ; et du CaCl₂ ;
dans lequel un changement de couleur de la composition, un changement de turbidité de la composition, ou les deux, lors de la mise en contact de l'échantillon avec la composition, indique la présence d'activité protéase dans l'échantillon,
**caractérisé en ce que** le procédé comprend en outre, avant la mise en contact de l'échantillon avec la composition, la préparation de la composition par :
réduction de l'acide tétrachloroaurique avec l'acide isoascorbique afin d'obtenir une dispersion des nanoparticules d'or dans laquelle les nanoparticules d'or sont non coiffées ;
enrobage des nanoparticules d'or dans la dispersion de nanoparticules d'or avec la gélatine, afin d'obtenir une dispersion de nanoparticules d'or enrobées ; et ensuite
addition du CaCl₂ à la dispersion de nanoparticules d'or enrobées.

2. Procédé selon la revendication 1, dans lequel la gélatine enrobant la surface des nanoparticules d'or est une gélatine non modifiée.

3. Procédé selon la revendication 1, dans lequel la composition ne comprend pas un agent dispersant.

4. Procédé selon la revendication 1, dans lequel l'activité protéase de l'échantillon est l'activité protéase d'une métalloprotéase matricielle.

5. Procédé selon la revendication 1, dans lequel le changement de couleur indique la présence d'activité protéase dans l'échantillon, de préférence dans lequel le changement de couleur est un changement de couleur du rouge vers le gris.

6. Procédé selon la revendication 1, dans lequel le changement de turbidité indique la présence d'activité protéase dans l'échantillon.

7. Procédé selon la revendication 1, dans lequel à la fois le changement de couleur et le changement de turbidité indiquent la présence d'activité protéase dans l'échantillon.

8. Procédé selon la revendication 1, dans lequel (i) le changement de couleur, le changement de turbidité, ou les deux, se produisent en l'espace d'une heure après la mise en contact de l'échantillon avec la composition ; ou :
dans lequel (ii) le changement de couleur, le changement de turbidité, ou les deux, se produisent en l'espace de trois minutes après la mise en contact de l'échantillon avec la composition.

9. Procédé selon la revendication 1, dans lequel l'activité protéase de l'échantillon est l'activité protéase d'une élastase des neutrophiles humains.

10. Procédé selon la revendication 1, qui ne détecte aucune protéase enzymatiquement inactive dans l'échantillon.

11. Procédé selon la revendication 1, dans lequel l'échantillon est un fluide d'une plaie humaine, de préférence dans lequel l'activité protéase de l'échantillon est l'activité protéase d'une protéase d'origine bactérienne ou plusieurs.

12. Procédé selon la revendication 1, dans lequel l'échantillon est mis en contact avec la composition dans un récipient qui a subi un traitement de surface pour repousser l'adsorption de la dispersion de nanoparticules d'or dont la surface est enrobée de gélatine.

13. Procédé selon la revendication 1, dans lequel la réduction de l'acide tétrachloroaurique avec l'acide isoascorbique est réalisée à température ambiante.

14. Kit, comprenant :
un flacon contenant une composition comprenant du CaCl₂ et une dispersion de nanoparticules d'or dont la surface est enrobée d'une gélatine;
un capuchon configuré pour être fixé au flacon, comprenant un septum en caoutchouc; et
un dispositif d'échantillonnage configuré pour collecter un liquide d'une plaie.
